# EUROPEAN PATENT APPLICATION

(11) **EP 1 679 509 A1**
(43) Date of publication of application: **12.07.2006**
(21) Application number: 04791404.9
(22) Date of filing: 06.10.2004
(51) Int. Cl.: G01N 27/407

(54) **MULTISENSOR MICROCHIP WHICH IS USED TO MEASURE GAS FLOW, TEMPERATURE AND CONCENTRATION IN ORDER TO CONTROL COMBUSTION, PRODUCTION METHOD THEREOF AND USE OF SAME**

(30) Priority: 10.10.2003 ES 200302362
(71) Applicant: CONSEJO SUPERIOR DE INVESTIGACIONES CIENTIFICAS, 28006 Madrid (ES); Copreci S. Coop, 20550 Aretxabaleta (ES); GAS NATURAL SDG, S.A., E-08002 Barcelona (ES); Universidad de Barcelona, 08007 Barcelona (ES); Fagor Electrodomesticos S. Coop., 20500 Mondragon (Guipuzcoa) (ES)
(72) Inventor: SABATE VIZCARRA, N. Instit. de Micro. de Barcelona, 08193 Barcelona (ES); GRACIA TORTADES, I., Int. de Micro. de Barcelona, 08193 Barcelona (ES); CANE BALLART, C., Inst. de Micro. de Barcelona, 08193 Barcelona (ES); CERDA BELMONTE, J., Univ. de Barcelona, 08007 Barcelona (ES); CIRERA HERNANDEZ, Albert Universidad de Barcelona, E-08007 Barcelona (ES); MORANTE LLEONART, J.R. Univ. de Barcelona, 08007 Barcelona (ES); BERGANZO RUIZ, Javier Fagor Electrodomesticos, 20500 Mondragón (ES)
(74) Representative: Ungria Lopez, Javier
(86) International application number: PCT/ES2004/070080
(87) International publication number: WO 2005/036157

(57) **Abstract**

The invention relates to the design and production of a microelectronic chip comprising an integrated assembly of gas flow and temperature sensors. The inventive microchip can be used to determine the concentrations of different gases in an environment, as well as the flow rates and temperatures thereof. The aforementioned measurements can be used to monitor combustion in a household furnace and to adjust the operation thereof in order to obtain safe, low-emission combustion.

## Description

### SECTOR OF THE ART

The invention comes within the low-cost instrumentation electronics sector based on the use of microelectronic technologies and microsystems. The application of the gas-detector multisensor microchip that is developed is aimed primarily at the sector of electronics for household applications, in the domestic appliance range.

### STATE OF THE ART

In recent years, the interest of producers of household boilers has been focused on improving the combustion quality, in other words, on their energy efficiency and their environmental impact. In spite of the efforts that have been invested, there currently exist two aspects involved in combustion control, which have a major influence on its quality, and with regard to which no action has yet been taken: the difference between the specific operating conditions of each facility and the variation in the composition of the supplied gas used in the combustion. The lack of optimisation of combustion on the basis of these parameters is the cause of the existence of undesired emissions, primarily CO. The determination of the oxygen (O₂) content together with residual carbon monoxide can be used to evaluate the combustion efficiency. In addition, the detection of levels of nitrous oxides (NOx) is a good indicator of the quality of the boiler in terms of the production of undesired emissions. In this regard, the implementation of a multifunctional system that would permit regulation and control of the parameters involved in the combustion, made up of an assembly of gas sensors, is of great interest.

So that the detection of gas concentrations can be useful and accurate in order to control combustion in a boiler, it is necessary to have an adequate knowledge of the working conditions of the sensors. Owing to the fact that the concentration measurement takes place in a circulating gas, the monitoring of its flow rate is of particular relevance. So, it becomes necessary to have a flow sensor in series with the assembly of gas concentrations sensors. Moreover, both the sensitivity of the flow sensors and that of the gas sensors depends on their working temperature and the circulating gas. For that reason, the implementation of a temperature sensor in the microsystem provides information on the temperature of the gases that are going to be measured.

The family of sensors that are usually developed for gas detection and which are suitable for inclusion in a chip are those based on a layer of metal oxides (MOS), such as SnO₂, WO₃, TiO₂, etc., obtained via various chemical synthesis routes or via physical deposition methods. Their detection mechanism is based on the change in the potential barrier of the oxide once exposed to the presence of the gas it is wished to measure. This produces a change in the resistivity of the material which is detected by means of a pair of electrodes located just below the sensor material, which can be deposited by means of techniques based on microdropping, screen-printing, spray, sputtering, etc.

Although traditionally these materials started to be deposited on the micromachined substrates in the form of a thin film (sputtering), innovations in synthesis and deposition techniques of sensitive materials permit microdrops to be obtained which imply an improvement in the sensitivity of the devices. The sensitivity of these materials to a certain gas depends mainly on their stoichiometric composition and on their working temperature.

In order for the detection phenomena to take place, the sensitive material has to be heated to a working temperature of typically between 200-550°C, depending on the composition of the oxide and on the type of gas to detect. So, for the correct functioning of these sensors, a heating element needs to be implemented which usually consists of a platinum or polysilicon resistor permitting the gas-sensitive material to be heated to the required working temperature, along with a pair of platinum electrodes which provide a reading of the change in resistivity of it. In order to prevent their electrical interaction, when the gas sensor device is produced, these two types of element are located at two different levels.

The combination of different gas sensor structures on a single chip permits the detection of different kinds of gas present in a particular atmosphere or the elimination of possible crossed sensitivities for a particular gas. In this regard, gas sensors based on metal oxides intended for CO detection are very sensitive to the presence of oxygen. In this way, owing to the fact that the gases resulting from partial combustion can include both types of gas, the concentration of them both needs to be evaluated simultaneously.

Among all the possible ways of measuring flow rates, the use of a principle based on heat transfer is the one most compatible with production technology for gas sensors. In particular, calorimetric type flow sensors based on three resistive elements are the most suitable since, as well as providing a very high sensitivity to the flow rate of gas in a bidirectional way, they can be implemented simultaneously into gas sensors if certain technological aspects are borne in mind.

The operating principle of this type of sensor is based on heating a resistive element to a temperature between 100-200°C above ambient temperature. Under static conditions, this resistor creates a symmetric temperature distribution around itself. This symmetry is altered in the presence of a circulating flow. The asymmetry generated in the temperature distribution by a gas flow can be detected if a sensor resistor is located on each side of the heating resistor, since the heat dissipated by the latter is transported in the direction of the flow by the effect of forced convection. So, the temperature difference between the sensor resistors is a parameter directly related to the flow rate of gas.

In order to implement this type of sensor in a chip, a single level of temperature-sensitive material is required that will permit the three resistors to be defined.

This patent describes the development of a multisensor microchip or microsystem composed of sensor elements based on microelectronic technology. This technology permits integration of low-cost sensor elements (batch mode production) and with high performance, an aspect that will facilitate their implementation in different industrial applications.

Finally, the adaptation of a measuring system to the needs of the application in a boiler means that the sensors have to be submitted to the same flow of gas coming from the smoke outlet. The hybrid integration of an assembly of sensors encapsulated on a unitary basis and connected in series is one possible solution, but it is not very suitable for implementation in a mass consumption market. It is therefore proposed to integrate the assembly of sensors into a single chip.

### Bibliography of Gas sensors

**Development of a micromachined hazardous gas sensor array.** *Sensors and Actuators B: Chemical , Volume* 93, *Issues 1-3, 1 August 2003, Pages 92-*99. Kraig D. Mitzner, Jason Sternhagen and David W. Galipeau.

**Miniaturised arrays of tin oxide gas sensors on single microhotplate substrates fabricated by micromoulding in capillaries**. *Sensors and Actuators B: Chemical , Volume* 93, *Issues 1-3, 1 August 2003, Pages 100-106*. Martin Heule and L. J. Gauckler.

**Micromechanical fabrication of robust low-power metal oxide gas sensors**. *Sensors and Actuators B: Chemical, Volume* 93, *Issues* 1-3, *1 August 2003, Pages* 345-349. Alois Friedberger, P. Kreisl, E. Rose, G. Müller, G. Kühner, J. Wöllenstein and H. Böttner.

**CO sensing with SnO**_{**2**} **thick film sensors: role of oxygen and water vapour**. *Thin Solid Films , Volume* 436, *Issue 1, 22 July 2003, Pages 17-24.* S. H. Hahn, N. Bärsan, U. Weimar, S. G. Ejakov, J. H. Visser and R. E. Soltis.

**DRIFT studies of thick film un-doped and Pd-doped SnO**_{**2**} **sensors: temperature changes effect and CO detection mechanism in the presence of water vapour**. *Thin Solid Films , Volume* 436, *Issue 1, 22 July 2003, Pages* 76-83. S. Harbeck, A. Szatvanyi, N. Bårsan, U. Weimar, and V. Hoffmann.

**Low-voltage and low-power optimization of micro-heater and its on-chip drive circuitry for gas sensor array**. *Sensors and Actuators A: Physical, Volume 100, Issue 1, 15 August 2002, Pages 94-101.* Yaowu Mo, Yuzo Okawa, Koji Imoue and Kazaki Natukawa.

**Thermal isolation in microsystems with porous silicon**. *Sensors and Actuators A: Physical, Volume* 99, *Issues 1-2, 30 April 2002, Pages 13-24*. V. Lysenko, S. Périchon, B. Remarki and D. Barbier.

**Gas sensing through thick film technology**. *Sensors and Actuators B: Chemical, Volume 84, Issue 1, 30 April 2002, Pages 72-77.* V. Guidi, M. A. Butturi, M. C. Carotta, B. Cavicchi, M. Ferroni, C. Malagu, G. Martinelli, D. Vincenzi, M. Sacerdoti and M. Zen.

**Thermal and gas-sensing properties of a micromachined thermal conductivity sensor for the detection of hydrogen in automotive applications**. *Sensors and Actuators A: Physical, Volumes* 97-98, *1 April 2002, Pages 104-108*. Isolde Simon and Michael Arndt.

**Wavelet transform and fuzzy ARTMAP-based pattern recognition for fast gas identification using micro-hotplate gas sensor**. *Sensors and Actuators B: Chemical, Volume 83, Issues 1-3, 15 March 2002, Pages 238-244.* E. Llobet, J. Brezmes, R. lonescu, X. Vilanova, S. Al-Khalifa, J. W. Gardner, N. Bârsan and X. Correig.

### Bibliography of Flow sensors

**A smart wind sensor using thermal sigma-delta modulation techniques,** Kofi A. A. Makinwa and Johan H. Huijsing Sensors and Actuators A: Physical, Volumes 97-98, 1 April 2002, Pages 15-20.

**Stainless steel-based integrated mass-flow controller for reactive and corrosive gases**, Kaoru Hirata and Masayoshi Esashi, Sensors and Actuators A: Physical, Volumes 97-98, 1 April 2002, Pages 33-38.

**A robust flow sensor for high pressure automotive applications**, U. Schmid, Sensors and Actuators A: Physical, Volumes 97-98, 1 April 2002, Pages 253-263.

**A silicon mass flow control micro-system**, Lieh-hsi Lo, Ming-jye Tsai, Tsung-han Tsai, Chin-Hon Fan, Chia-lin Wu and Ruey-shing Huang. Mécanique & Industries, Volume 2, Issue 4, July-August 2001, pages 363-369.

**A silicon flow sensor for gases and liquids using AC measurements,** Gerlinde Bedõ, Heike Fannasch and Rudolf Müller, Sensors and Actuators A: Physical, Volume 85, Issued 1-3, 25 August 2000, Pages 124-132.

**Novel C-MOS compatible monolithic silicon gas flow sensor with porous silicon thermal isolation**, G. Kaltass and A. G. Nassiopoulou, Sensors and Actuators A: Physical, Volume 76, Issues 1-3, 30 August 1999, Pages 133-138.

**Thermal flow sensor for liquids and gases based on combinations of two principles,** M. Ashauer, H. Glosch, F. Hedrich, N. Hey, H. Sandmaier and W. Lang, Sensors and Actuators A: Physical, Volume 73, Issues 1-2, 9 March 1999, Pages 7-13.

**The determination of the effective ambient temperature for thermal flow sensors in a non-isothermal environment**, B. W. van Oudheusden, Sensors and Actuators A: Physical, Volume 72, Issue 1, 8 January 1999, Pages 38-45.

**A conduction-convection design for liquid flow sensing**, L. Castañer, V. Jiménez, F. Masana, M. Dominguez and A. Rodríguez, Sensors and Actuators A: Physical, Volume 66, Issues 1-3, 1 April 1998, Pages 131-137.

**A polysilicon flow sensor for gas flow meters**, T. Neda, K. Nakamura and T. Takumi, Sensors and Actuators A: Physical, Volume 54, Issues 1-3, June 1996, Pages 626-631.

**A high sensitivity CMOS gas flow sensor on a thin dielectric membrane,** David Moser and Henry Baltes, Sensors and Actuators A: Physical, Volumes 37-38, June-August 1993, Pages 33-37.

**Micro-liquid flow sensor**, Theo S. J. Lammerink, Niels R. Tas, Miko Elwenspoek and Jan H. J. Fluitman, Sensors and Actuators A: Physical, Volumes 37-38, June-August 1993, Pages 45-50.

### Bibliography of Multisensor integration

**Integrated comfort sensing system on indoor climate**, Jeongho Kang and Sekwang Park, Sensors and Actuators A: Physical, Volume 82, Issues 1-3, 15 May 2000, Pages 302-307.

**Multi-parameter detection in fluid flows**, J. van Kuijk, T. S. J. Lammerink, H. E. de Bree, M. Elwenspoek and J. H. J. Fluitman, Sensors and Actuators A: Physical, Volume 47, Issues 1-3, March-April 1995, Pages 369-372.

**A micromachined pressure/flow-sensor**, R. E. Oosterbroek, T. S. J. Lammerink, J. W. Berenschot, G. J. M. Krijnen, M. C. Elwenspoek and A. van de Berg, Sensors and Actuators A: Physical, Volume 77, Issue 3, 2 November 1999, Pages 167-177.

### DESCRIPTION OF THE INVENTION

### Brief description of the invention

An object of the present invention consists of a multisensor microchip or microsystem which includes on a single silicon matrix various gas sensors of the semiconductor type, a flow sensor of the thermal type and a resistive temperature sensor, which is created by standard technology and has specific characteristics, together with its production method. This microchip, produced by means of the compatibilisation of the production technologies of different sensors, permits monitoring of gases coming from combustion in order to generate an action permitting that combustion to be optimised. This multisensor microchip can be used in the production of gas detection devices for applications concerning the determination of gas concentration, flow and temperature in a particular atmosphere, for use in the monitoring and control of combustion in, for example, household and industrial heaters and boilers.

### Detailed description of the invention

The present invention is based on the fact that the Inventors have observed that the detection of different kinds of gas present in a single atmosphere, together with the measurement of other parameters such as flow and temperature, is possible by means of the compatibilisation of the different production technologies of the sensors of those parameters on a single multisensor microchip or microsystem. Specifically, the compatibilisation of production technologies for gas sensors of the semiconductor type based on silicon substrates micromachined with the production technology of calorimetric flow sensors.

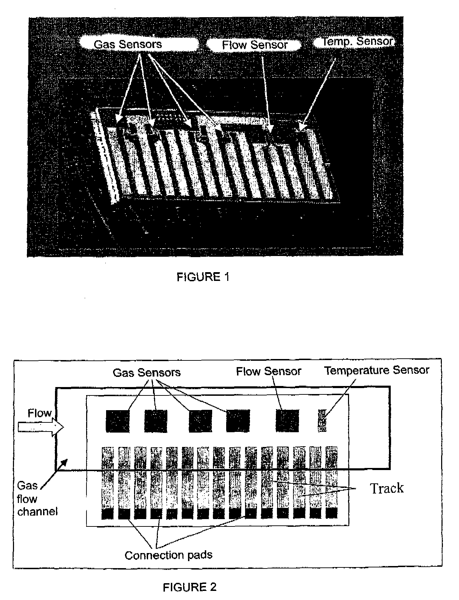

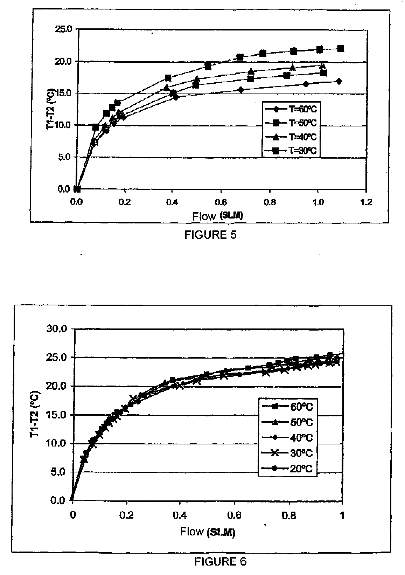

So, an object of the present invention consists of a multisensor microchip or microsystem, hereinafter the inventive multisensor microchip, which includes on a single silicon matrix various gas sensors of the semiconductor type, a flow sensor of the thermal type and a resistive temperature sensor, which is created by standard technology and has the following specific characteristics:
a) the gas and flow sensors are compatibilised in a single level of the microchip (Figure 5) thanks to the fact that one of the materials used for the gas sensors is used in the implementation of the flow sensor, specifically platinum,
b) the sensors are produced on individual dielectric membranes totally secured via their sides permitting optimum thermal isolation of the resistors at the same time as low power consumption,
c) the dielectric membranes defining the different sensors are situated at one end and not in the middle, with the aim of keeping the sensor areas of the connection pads as far apart as possible, in such a way that permits a flow channel to be defined which covers solely the gas-sensitive zone while the connection pads are kept outside of it as shown in Figure 2,
d) the different sensors are arranged in such a way that the gas flow to monitor interacts with them in the following order: gas sensors, flow rate sensor and gas temperature sensor, and
e) optionally, said multisensor microchip can contain integrated into it the electronics for the polarisation of the heaters and the amplification and linearisation of the output signals from the sensors.

A particular object of the present invention consists of an inventive multisensor microchip in which the gases to identify are O₂, CO at low concentrations, CO at high concentrations, and NOₓ.

Another particular object of the present invention consists of an inventive multisensor microchip designed for operating in open and closed environments. A particular embodiment of an inventive multisensor microchip consists of a microchip for operating in closed environments, for the specific application of controlling the combustion gases in the outlet duct from a boiler, incorporating an encapsulating on the multisensor microchip which defines a duct of fixed cross-section and which permits the determination of the flow rate of those gases. Another particular inventive embodiment consists of a microchip adapted to open environments which would not require the flow sensor but for which the gas detection would be carried out without any problem.

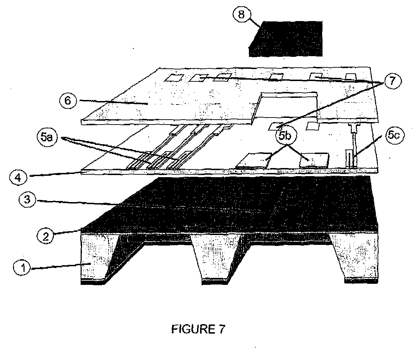

The production method for the multisensor microchip is based on standard microelectronic technology stages on silicon substrates and additional techniques of micromachining, which provide the necessary thermal isolation so that the sensors, which operate at high temperatures (200-550°C), can have a low power consumption (see Example 1). During the inventive production method the preparation of the gas sensors requires two levels of resistive material: polysilicon and platinum. The polysilicon is used in the implementation of the heating resistor permitting the gas-sensitive material to be heated (Points 3 and 5 in Figure 7), while the platinum is used both for defining the electrodes of the gas sensor and for making electrical contact with the polysilicon level, thereby replacing the typical aluminium used in a standard microelectronic process (for example, CMOS: Complementary Metal Oxide Semiconductor).

As mentioned above, owing to the fact that the flow sensor requires a single level of temperature-sensitive material, its compatibilisation with the gas sensor requires that one of the materials used for the gas sensors should be used in the implementation of the flow sensor. In this regard, platinum, which is a highly stable material, with a linear behaviour in a wide temperature range and a temperature coefficient of resistance (TCR) greater than polysilicon, is the ideal candidate among the noble metals for integration; another potential noble metal as an alternative to platinum is gold.

So, a further object of the present invention consists of an inventive production method for the multisensor microchip, hereinafter referred to as the inventive production method, based on standard microelectronic technology stages on silicon substrates and with the following own characteristics:
i) the membranes of the device, produced by means of a stage of volume micromachining of the silicon, have been optimised for use in flow microsensors of the thermal type and gas microsensors of the semiconductor type, since they provide the necessary thermal isolation so that the sensors can operate at high temperatures (200-550°C) with low power consumption,
ii) the preparation of the gas sensors requires two levels of material: polysilicon and platinum, in such a way that the polysilicon is used in the implementation of the heating resistor permitting the gas-sensitive material to be heated (Points 3 and 5 of Figure 7), while the platinum is used both for defining the electrodes of the gas sensor and for making electrical contact with the polysilicon level, thereby replacing the typical aluminium used in a standard microelectronic process (for example, CMOS: Complementary Metal Oxide Semiconductor),
iii) the flow and temperature sensors, which require a single level of temperature-sensitive material, are developed and produced in a way that is compatible with the gas sensor, which requires that one of the materials used for the gas sensors should be used in the implementation of the flow sensor, for example, materials with high stability, linear behaviour in a wide temperature range and a temperature coefficient of resistance (TCR) greater than polysilicon, such as the noble materials platinum and gold, and
iv) in addition, and in applications requiring it, the multisensor can be passivised with a SiO₂/Si₃N₄ bilayer, which protects the sensor elements from the degradation that can be caused by the presence of humidity or traces of corrosive gases.

Another object of the present invention consists in the use of the inventive method for the preparation and production of the inventive multisensor microchip.

Finally, another object of the present invention consists of the use of the inventive multisensor microchip in the production of gas detection devices for applications involving the determination of the gas concentration, flow and temperature in a particular atmosphere, for use in the monitoring and control of the combustion; such as for example, and solely by way of illustration without affecting the inventive scope, household and industrial heaters or boilers.

The characteristics of the inventive multisensor microchip will be able to be better understood with the present description made with reference to the attached drawings, in which a non-limiting example of embodiment is shown.

### DETAILED DESCRIPTION OF THE DRAWINGS

**Figure 1**: Photograph of the produced microsystem.
**Figure 2**: Diagram of the arrangement of the chip in a flow channel.
**Figure 3**: Response of the gas sensors implemented in the multisensor microchip to a change in the CO concentration of up to two orders of magnitude. Sensor A corresponds to the measurement device for CO at high concentrations, sensors B and C correspond to devices simultaneously determining low concentrations of CO and of oxygen, and sensor D is the measurement device for NOx.
**Figure 4**: Characteristic curve of one of the gas-sensitive sensors to the change of air flow demonstrating the need for joint implementation of the different sensors.
**Figure 5**: Characteristic response curve of the sensor to different temperatures, prior to its temperature compensation with conventional external electronics.
**Figure 6**: Characteristic response curve of the sensor to different temperatures, after its temperature compensation with conventional external electronics.
**Figure 7:** 3D diagram of the structure of the multisensor microchip separated into the parts forming it. Identification of the parts of the microsystem: 1, Silicon substrate; 2, Dielectric layer; 3, Polysilicon heating resistor; 4, Layer of interlevel oxide; 5, Level of platinum which defines: a) Sensor resistors for detection of the air flow; b) Electrodes for material sensitive to gas concentration; and c) Sensor resistor for temperature; 6, Passivation dielectric layer; 7, Opening of contacts; 8, Gas-sensitive material.
**Figure 8**: SEM images of a microdrop of SnO₂: (a) View of the microdrop in cross-section, b) Aerial view of the microdrop deposited on the micromachined substrate.

### EXAMPLE OF EMBODIMENT OF THE INVENTION

### Example 1.- Production method of the multisensor microchip

The production method for the inventive multisensor microchip is described based on the simplified 3D diagram (with just one sensor of each type) of the structure of the microsystem shown in Figure 7. On the basis of a silicon wafer (1), polished on both sides, a dielectric layer (2) is grown consisting of 1000 A of SiO₂ and 3000 A of Si₃N₄. Defined on this dielectric layer are the polysilicon heating resistors (3) which will permit heating of the material sensitive to the gas sensors to the working temperature. A layer of 5500 A of SiO₂ is then deposited which permits the polysilicon to be electrically isolated from the layer of platinum which is deposited next (4). Defined on this metal layer are different elements: the sensor resistors forming the flow sensor (7-a), the electrodes on which the gas-sensitive material is deposited (7-b) and the sensor resistors for temperature (7-c). A passivation dielectric layer (6) protects these elements from corrosion in certain atmospheres, thus considerably extending the mean life of the devices. Some openings (7) are made on this layer which permit the electrical contacts of the different sensitive elements to be established.

Moreover, and with the aim of obtaining the thermal isolation required in this type of sensor, an anisotropic etching of the silicon substrate is carried out. This stage, known as micromachining of the silicon, permits the dielectric membranes to be defined beneath the zones where the sensor resistors corresponding to the flow sensor and the heating resistors of the gas sensors have previously been defined.

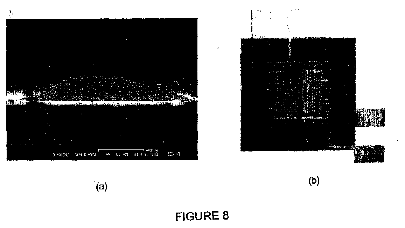

Finally, once the multisensor chip has been obtained, the materials sensitive to the different gases it is wished to detect are then deposited on the electrodes. Figure 8 shows two details, with a side and upper view, of one of the gas sensors of the multisensor chip with sensitive material deposited in the form of microdrops on one of the micromachined membranes (change of site).

The dimensions of the membranes defining the structure of the different sensors are 1000 x 1000 µm. The active zone of the gas sensors is 500 x 500 µm. The total dimensions of the chip are 12000 x 6000 µm².

The production of the microsensor chip is carried out by means of microelectronic processes on silicon substrates and additional micromachining techniques, which provide the necessary thermal isolation so that the sensors, which operate at high temperatures (200-550°C), can have a low power consumption.

This solution does not just present advantages in the simplification of the encapsulating; it also increases the performance of the assembly on account of the proximity of the different sensors and it reduces the area of silicon needed for its production, thereby reducing its unit cost always provided a production method is designed with a sufficiently high output. The combined integration of the sensors entails the development of a suitable technology and an adequate design.

The multisensor microchip is developed so that its chip-on-board type implementation, together with the control electronics and the necessary reading, is possible.

### Example 2.- Measurement of the ratio of inlet air and exhaust gas concentration of a household boiler for combustion control.

As an example of application of the invention of this patent, explained below is the use of the inventive multisensor microchip for controlling the combustion of a household boiler with the aim of optimising its efficiency and minimising undesired emissions.

The efficiency in the combustion of a boiler is given by the ratio of inlet air / methane gas, also known as the lambda parameter. Within a narrow range of values of this parameter (1.2-1.5, depending on the type and quality of gas), the energy efficiency of the combustion is maximum. In other words, methane gas is entirely consumed with the minimum necessary inlet air (an excess of air would permit complete combustion of the methane but would reduce the energy efficiency of the boiler).

Outside of that range, the efficiency of the combustion drops, and undesired emissions also appear, basically NOₓ and CO.

Currently, household boilers incorporate the necessary electronics for controlling both the fan and the gas valve. Nevertheless, this control is programmed during calibration in factory and so, once installed, the boilers do not always work under optimum conditions in terms of environmental impact or energy efficiency because other factors beyond control come into play in the combustion. Included among these factors are the variable and specific operating conditions deriving from each facility or gas composition, as well as factors of ageing and climatic conditions, among others.

The measurement of O₂ and CO at the outlet from the boiler permits one to learn the quality of the combustion, since CO emissions imply the existence of an incomplete combustion of the gas while an increase in O₂ reveals an excess of air in the inlet. The control strategy that is implemented is based on regulation of the inlet air for the boiler (via the speed of the fan) and of the methane (via the gas valve) depending on the concentration of these two gases at the outlet from the boiler. It is also of interest from the point of view of environmental safety to detect other gases of the NOₓ type, which are highly reactive, and to detect high concentrations of CO, which can be harmful for human beings.

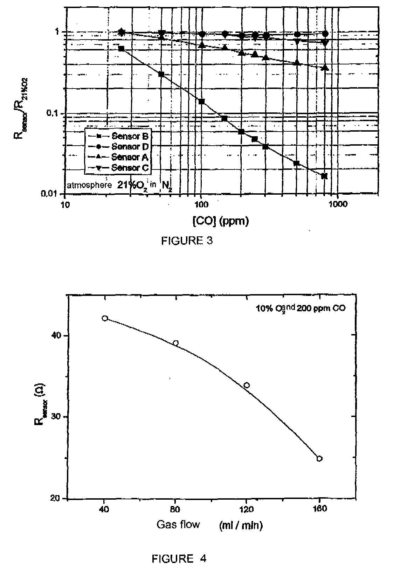

So, the multisensor microchip described in this patent permits concentrations of O₂, CO (at low and high concentrations) and of NOₓ to be measured. Figure 3 shows the response of the four gas sensors implemented in the microchip to a variation in the concentration of CO of up to two orders of magnitude. Moreover, the detection of the different gases is carried out in the flow of gases circulating in the pipe of the gases coming from the combustion, due to which the flow is a parameter that influences the response of the sensors. Figure 4 shows how the response of a sensor varies when the flow rate of the gas to measure is altered. This fact demonstrates the need to implement a flow sensor in the multisensor microchip in order to permit the variation of the signal due to this effect to be corrected.

Likewise, owing to the temperature dependence of the response from the flow sensor and of the gas sensors, the microchip also includes a temperature sensor consisting of a platinum resistor which permits the temperature of the exhaust gases to be monitored and to correct the signal from the flow sensor. Figures 5 and 6 show the characteristic response curves of the flow sensor to different temperatures before and after compensation of the signals with conventional external electronics.

The gas sensors incorporated into the multisensor microchip can operate in open environments. Nevertheless, for the specific control application of combustion gases in the outlet duct of a boiler, which will also take into account the flow of those gases, it is necessary to incorporate encapsulating on the multisensor microchip which will define a duct of fixed cross-section. The encapsulating consists of a piece of plastic which fixes a cross-section of air above the sensor of 7 mm², in order to achieve good functioning of the different sensors. This encapsulating can consist of different materials, plastic or metal, according to the working temperature at which it has to operate.

## Claims

1. Multisensor microchip for gases **characterised in that** it includes on a single silicon matrix various gas sensors of the semiconductor type, a flow sensor of the thermal type and a resistive temperature sensor, which is created by standard technology and has the following specific characteristics:
a) the gas, flow and temperature sensors are compatibilised in a single level of the microchip (Figure 5) thanks to the fact that one of the materials used for the gas sensors is used in the implementation of the flow sensor, specifically platinum,
b) the sensors are produced on individual dielectric membranes totally secured via their sides permitting optimum thermal isolation of the resistors at the same time as low power consumption,
c) the dielectric membranes defining the different sensors are situated at one end and not in the middle, with the aim of keeping the sensor areas of the connection pads as far apart as possible, in such a way that permits a flow channel to be defined which covers solely the gas-sensitive zone while the connection pads are kept outside of it (Figure 2),
d) the different sensors are arranged in such a way that the gas flow to monitor interacts with them in the following order: gas sensors, flow rate sensor and gas temperature sensor, and
e) optionally, said multisensor microchip can contain integrated into it the electronics for the polarisation of the heaters and the amplification and linearisation of the output signals from the sensors.

2. Multisensor microchip according to claim 1, **characterised in that** the gases to identify are O₂, CO at low concentrations, CO at high concentrations, and NOₓ.

3. Multisensor microchip according to claim 1, characterised for operating in open and closed atmospheres.

4. Multisensor microchip according to claim 3, **characterised in that** it is a microchip for operating in closed environments, consisting of an encapsulating on the multisensor microchip which defines a duct of fixed cross-section and which permits the determination of the flow rate of those gases.

5. Production method of the multisensor microchip according to any of claims 1 to 4 based on standard microelectronic technology stages on silicon substrates **characterised by** the following own specificities:
i) the additional techniques of micromachining provide the necessary thermal isolation so that the sensors which operate at high temperatures (200-550°C) can have low power consumption (see Example 1),
ii) the preparation of the gas sensors requires two levels of material: polysilicon and platinum, in such a way that the polysilicon is used in the implementation of the heating resistor permitting the gas-sensitive material to be heated (Figure 3 and 5), while the platinum is used both for defining the electrodes of the gas sensor and for making electrical contact with the polysilicon level, thereby replacing the typical aluminium used in a standard microelectronic process (for example, CMOS: Complementary Metal Oxide Semiconductor),
iii) the flow sensor and the temperature sensor, which require a single level of temperature-sensitive material, are developed and produced in a way that is compatible with the gas sensor, which requires that one of the materials used for the gas sensors should be used in the implementation nf the flow sensor, for example, materials with high stability, linear behaviour in a wide temperature range and a temperature coefficient of resistance (TCR) greater than polysilicon, such as the noble materials platinum and gold, and
iv) in addition, and in applications requiring it, the multisensor can be passivised with a SiO₂/Si₃N₄ bilayer, which protects the sensor elements from the degradation that can be caused by the presence of humidity or traces of corrosive gases.

6. Use of the method according to claim 5 for the preparation and production of the inventive multisensor microchip according to any of claims 1 to 4.

7. Use of the method according to any of claims 1 to 4 in the production of gas detection devices for applications involving the determination of the gas concentration, flow and temperature in a particular atmosphere, for use in the monitoring level of emissions and control of the combustion.

8. Use of the multisensor microchip according to claim 7 **characterised in that** the device or apparatus belongs to, among others, the following group: household heaters or boilers.
